# EUROPEAN PATENT SPECIFICATION

(11) **EP 3 248 614 B1**
(45) Date of publication and mention of the grant of the patent: **21.05.2025**
(21) Application number: 17178151.1
(22) Date of filing: 22.12.2011
(51) Int. Cl.: A61K 39/02, C12N 1/36, A61K 8/20, A61Q 19/08, A61K 38/48, A61K 8/66, A61K 8/29, A61Q 19/00, A61P 25/06, A61P 25/00, A61P 21/00, A61P 19/02, A61P 17/10, A61P 17/00, A61P 1/00, A61K 8/02

(54) **KITS FOR TOPICAL APPLICATION, REMOVAL, AND INACTIVATION OF THERAPEUTIC OR COSMETIC BOTULINUM TOXIN COMPOSITIONS**
KITS ZUR TOPISCHEN ANWENDUNG, ENTFERNUNG UND INAKTIVIERUNG THERAPEUTISCHER ODER KOSMETISCHER BOTULINUS TOXIN ZUSAMMENSETZUNGEN
KITS POUR APPLICATION TOPIQUE, RETRAIT ET INACTIVATION DE COMPOSITIONS DE TOXINES BOTULINIQUES THÉRAPEUTIQUES OU COSMÉTIQUES

(30) Priority: 07.01.2011 US 201161430868 P
(43) Date of publication of application: 29.11.2017
(62) Divisional of application: 11855190.2
(73) Proprietor: ReVance Therapeutics, Inc., Newark, CA 94560 (US)
(72) Inventor: WAUGH, Jacob, San Francisco, CA California 94105 (US); RUEGG, Curtis, Redwood City, CA California 94062 (US)
(74) Representative: J A Kemp LLP

(56) References cited:
- WO-A1-2008/109252
- WO-A2-2006/094263
- DE-A1- 102006 019 447
- US-A- 5 624 636
- US-A1- 2008 220 021
- US-A1- 2009 148 342
- US-A1- 2010 330 123
- SENOGLES P-J ET AL: "Photocatalytic Degradation of the Cyanotoxin Cylindrospermopsin, using Titanium Dioxide and UV Irradiation", WATER RESEARCH, ELSEVIER, AMSTERDAM, NL, vol. 35, no. 5, 1 April 2001 (2001-04-01), pages 1245 - 1255, XP004314801, ISSN: 0043-1354, DOI: 10.1016/S0043-1354(00)00372-9

## Description

This application claims the benefit of priority under 35 U.S.C. § 119 to U.S. Provisional Patent Application No. 61/430,868, filed January 7, 2011.

### FIELD OF THE INVENTION

This invention relates to kits for safely applying, removing, and inactivating topical therapeutic or cosmetic toxin compositions.

### BACKGROUND OF THE INVENTION

Botulinum toxin (also known as botulin toxin or botulinum neurotoxin) is one of the most potent naturally occurring neurotoxins known to man. It produces paralysis of muscles by preventing synaptic transmission or release of acetylcholine across the neuromuscular junction, and is thought to act in other ways as well. Botulinum toxin essentially blocks signals that normally would cause muscle spasms or contractions, resulting in paralysis.

Despite its toxicity, botulinum toxin has found numerous therapeutic and cosmetic uses. For example, botulinum toxin has been used to treat a variety of conditions, including hemifacial spasm, adult onset spasmodic torticollis, anal fissure, blepharospasm, cerebral palsy, cervical dystonia, migraine headaches, strabismus, temporomandibular joint disorder, and various types of muscle cramping and spasms. In addition, the muscle-paralyzing effects of botulinum toxin have been used in therapeutic and cosmetic facial applications such as treatment of wrinkles, frown lines, and other results of spasms or contractions of facial muscles.

Conventionally, botulinum toxin is administered via injection to the area in need of treatment. Administration of botulinum toxin by injection typically involves reconstituting a previously lyophilized sample of botulinum toxin using saline or some other pharmaceutically acceptable diluent. The reconstituted botulinum toxin solution is then drawn into a syringe, where it remains until injection into the patient. The sequence of steps required for preparation and subsequent administration of the botulinum toxin is relatively safe for the clinician, as the reconstituted botulinum toxin solution is contained in either the syringe or a vial until administration. Furthermore, the doses of botulinum toxin used in the injectable formulation are also relatively low. For example, current injectable botulinum toxin formulations for treating wrinkles contain approximately 100 U of toxin per dose.

More recent advances have allowed for topical administration of botulinum toxin. See *e.g.,* U.S. Patent Application 11/072,026. However, the use of topical botulinum toxin formulations introduces new and unforeseen challenges for the clinician, particularly with respect to the safe application, handling and disposal of botulinum toxin. Accordingly, safe and effective means for applying, removing and inactivating excess botulinum toxin is needed.

US 2008/0107690 A1 discloses compositions and methods for topical application and transdermal delivery of botulinum toxins.

### SUMMARY OF THE INVENTION

The invention is defined by the claims. Further aspects and preferred embodiments are defined in the dependent claims. Any aspects, embodiments and examples of the present disclosure which do not fall under the scope of the appended claims do not form part of the invention and are merely provided for illustrative purposes.

The invention provides a kit for topically applying a botulinum toxin for transdermal delivery, the kit comprising:
a composition comprising botulinum toxin and an indicator to allow for monitoring the location and presence of the composition, further wherein the indicator is for indicating the area of skin that has been treated by the composition;
an applicator for topically applying the composition to an area in need of treatment;
a removal agent for removing excess toxin from the treated area, said removal agent comprising an agent that absorbs, adsorbs, or physically scrapes off the excess toxin; and
an inactivation agent for inactivating the excess toxin, thereby rendering the toxin without neurotoxicity.
One aspect of this invention is the recognition that topical toxin formulations, such as, for example, topical botulinum toxin formulations, must be handled and disposed of using different procedures compared to conventional injectable formulations containing the same toxins. In particular, the invention recognizes that undesirable contamination can occur without proper care and safe handling procedures, since topical toxin formulations can be inadvertently spread to other surfaces via casual contact or spillage.

Another aspect of the invention is the recognition that the toxin content in topical botulinum formulations may, in many instances, be significantly higher than that of injectable botulinum toxin formulations. Without wishing to be bound by theory, it is believed that a higher botulinum toxin content in topical formulations is necessary to achieve therapeutic or cosmetic effects, owing to the difficulty of transporting botulinum toxin through skin, even with carriers or skin-penetration enhancers, as described herein. For instance, a single dose of a topical botulinum toxin type A formulation may comprise more than 1000-7000 U of toxin, whereas an injectable formulation typically contains only about 100 U. Thus, the invention recognizes, for the first time, that significant amounts of botulinum toxin may remain on the skin surface after administration of a topical composition, thereby posing a potential hazard to patient and clinician alike and creating potential environmental issues.

Accordingly, one aspect of this invention is to provide kits for safely applying, removing and inactivating excess toxin, (e.g., botulinum toxin), used in topical therapeutic or cosmetic compositions.

The invention provides a kit for topically applying a botulinum toxin for transdermal delivery. The kit includes a composition comprising a botulinum toxin and an indicator to allow for monitoring the location and presence of the composition, further wherein the indicator is for indicating the area of skin that has been treated by the composition; and an applicator for topically applying the composition to an area in need of treatment. Optionally, the toxin composition may contain a dye. The kit also includes a removal agent for removing excess toxin from a treated area, said removal agent for removing excess toxin from the treated area, said removal agent comprising an agent that absorbs, adsorbs, or physically scrapes off the excess toxin, and an inactivation agent for inactivating the excess toxin thereby rendering the toxin without neurotoxicity.

The botulinum toxin may be any of botulinum toxin serotypes A, B, C₁, D, E, F, or G. In one particularly preferred embodiment, the botulinum toxin is purified botulinum toxin type A. In certain embodiments, the indicator may be a detectable dye, such as a visible dye or a fluorescent dye. In preferred embodiments, such detectable dyes, when used in the composition, do not react with the botulinum toxin. Other indicators are also contemplated by this invention, as described herein.

### DETAILED DESCRIPTION OF THE INVENTION

This invention provides kits for safely removing and inactivating topical toxin compositions. As used herein, the terms "inactivate", "inactivating", "inactivation", or variants thereof, when used in connection with a toxin, refers to treating the toxin to render it without neurotoxicity.

The invention also contemplates kits for both applying, removing and inactivating topical toxin compositions.

The topical toxin compositions are not particularly limited, and may comprise any toxin capable of producing a therapeutic or cosmetic benefit after being topically applied to a surface area of a subject's body. The topical toxin composition may comprise a toxin that acts a paralytic agent. Generally speaking, a paralytic agent may be any agent that can interrupt nerve impulse transmission across a neuromuscular or neuroglandular junction, block or reduce neuronal exocytosis of a neurotransmitter or alter the action potential at a sodium channel voltage gate of a neuron. Non-limiting examples of paralytic substances include botulinum toxins (including serotypes A, B, C₁, D, E, F, and G), tetanus toxins, saxitoxins, and tetrodotoxin, as well as combinations thereof.

The term "botulinum toxin" as used herein is meant to refer to any of the known types of botulinum toxin, whether produced by the bacterium or by recombinant techniques. The botulinum toxin may be obtained from any of the known serotypes of *C. botulinum (e.g.,* serotypes A, B, C₁, D, E, F, or G). In certain preferred embodiments, the botulinum toxin is present as an isolated botulinum toxin molecule (e.g., molecules of botulinum toxin types A, B, C₁, D, E, F, or G without the stabilizing proteins expressed by C. *botulinum).* In such embodiments, the isolated botulinum toxin molecule is preferably stabilized by exogenous stabilizers. See, *e.g.,* U.S. Provisional Application No. 61/220,433 entitled "Albumin Free Botulinum Toxin Formulations". Alternatively, the botulinum toxin may be present in a complexed form, stabilized, at least in part, by one or more of the non-toxin, non-hemaglutinin proteins and non-toxin, hemaglutinin proteins that are normally expressed along with the botulinum toxin by the C. *Botulinum* bacteria. In certain embodiments, the botulinum toxin is stabilized by exogenous stabilizers, such as albumin. The invention also specifically contemplates the use of commercially available botulinum toxin formulations, non-limiting examples of which include BOTOX^{®} Dysport^{®} and Xeomin^{®}

The botulinum toxin used in the invention can alternatively be a botulinum toxin derivative, that is, a compound that has botulinum toxin activity but contains one or more chemical or functional alterations on any part or on any chain relative to naturally occurring or recombinant native botulinum toxins. For instance, the botulinum toxin may be a modified neurotoxin (e.g., a neurotoxin which has at least one of its amino acids deleted, modified or replaced, as compared to a native, or a recombinantly produced neurotoxin or a derivative or fragment thereof). For instance, the botulinum toxin may be one that has been modified in a way that, for instance, enhances its properties or decreases undesirable side effects, but that still retains the desired botulinum toxin activity. The botulinum toxin may be any of the botulinum toxin complexes produced by the bacterium, as described above. Alternatively, the botulinum toxin may be a toxin prepared using recombinant or synthetic chemical techniques (e.g. a recombinant peptide, a fusion protein, or a hybrid neurotoxin, as prepared from subunits or domains of different botulinum toxin serotypes (see U.S. Pat. No. 6,444,209, for instance)). The botulinum toxin may also be a portion of the overall molecule that has been shown to possess the necessary botulinum toxin activity, and in such case may be used per se or as part of a combination or conjugate molecule, for instance a fusion protein. Additionally, the botulinum toxin may be in the form of a botulinum toxin precursor, which may itself be non-toxic, for instance a nontoxic zinc protease that becomes toxic on proteolytic cleavage.

Generally speaking, the kits provided herein for safely removing and inactivating topical therapeutic or cosmetic toxin compositions (e.g., botulinum toxin compositions) are suitable for use with any mode of topical administration of a therapeutic or cosmetic toxin composition, as discussed herein. The topical administration may be to any area of the body in need thereof. In certain preferred embodiments, the area of the body to be treated is selected from the group consisting of the face, the axilla, the palms of the hands, the hands, the feet, the lower back, the neck, the leg, the groin, the arm, the elbow, the knee, the pelvis, the buttocks and the torso.

In some embodiments, the kit contains the topical toxin composition administered to produce a cosmetic effect. For instance, botulinum toxin type A neurotoxin may be applied to the face to reduce the appearance of wrinkles, including marionette lines, nasolabial lines, crows feet, brow furrows, glabellar lines, and combinations thereof.

In other embodiments, the kit contains the topical toxin composition is administered to provide a therapeutic effect to a subject. For instance, the topical toxin composition may attenuate cholinergic nerve impulses, thereby suppressing output from a gland. In certain non-limiting embodiments, the topical toxin composition comprises botulinum toxin that is administered to reduce hypersecretion of sweat glands or sebaceous glands, in order to treat hyperhidrosis or acne, respectively. More generally, topical botulinum toxin compositions are administered to treat any indication for which botulinum toxin is known to provide an improvement in condition. Non-limiting examples of such indications include hemifacial spasm, adult onset spasmodic torticollis, anal fissure, blepharospasm, cerebral palsy, cervical dystonia, migraine headaches, strabismus, temperomandibular joint disorder, and various types of muscle cramping and spasms.

The compositions are applied so as to administer an effective amount of the toxin. The terms "effective amount" used herein refers an amount of a toxin (e.g., botulinum toxin) that is sufficient to produce the desired muscular paralysis or other biological or aesthetic effect, but that implicitly is a safe amount, i.e. one that is low enough to avoid serious side effects. Desired effects include the relaxation of certain muscles with the aim of, for instance, decreasing the appearance of fine lines and/or wrinkles, especially in the face, or adjusting facial appearance in other ways such as widening the eyes, lifting the corners of the mouth, or smoothing lines that fan out from the upper lip, or the general relief of muscular tension. The last-mentioned effect, general relief of muscular tension, can be effected in the face or elsewhere. In some embodiments, the compositions used in the kit of the invention may contain an appropriate effective amount of the botulinum toxin for application as a single-dose treatment, or may be more concentrated, either for dilution at the place of administration or for use in multiple applications. The botulinum toxin is administered topically for transdermal delivery to muscles or to other skin-associated structures. The administration may be made, for example, to the legs, shoulders, back (including lower back), axilla, palms, feet, neck, groin, dorsa of the hands or feet, elbows, upper arms, knees, upper legs, buttocks, torso, pelvis, or any other part of the body where administration of the botulinum toxin is desired.

In certain embodiments, the dose of botulinum toxin present in the topical toxin compositions used in the kit of the invention is in the range of 100 U to 7000 U, more preferably from 500 U to 6000 U, more preferably 1000 U to 5000 U, and even more preferably from 2000 to 4000 U. In embodiments, the botulinum toxin dose is in the range of 1000 to 7000 U, although the invention specifically contemplates that the lower and/or upper limits of this range may be increased or decreased, respectively, by intervals of 50 U, with each sub-range considered to be an embodiment of the invention.

The topical toxin composition may further include an indicator that permits a clinician to see the topical toxin composition more easily. Thus, for example, in embodiments wherein the clinician manually applies the topical botulinum toxin formulation directly to the area to be treated, the indicator may aid the clinician in determining which areas of skin already have been treated. In addition, the indicator may allow the clinician to easily spot areas that have been contaminated by the topical toxin composition. Generally speaking, the indicator is inert with respect to the toxin and may be safely applied to the surface of the skin at the concentration used. In certain preferred embodiments, the indicator is a detectable dye. The dye may be a visible or fluorescent dye that is produced from natural sources or produced artificially. Dyes from natural sources may include, but are not limited to, natural food dyes. Non-limiting examples of visible dyes include, but are not limited to, FD&C Blue No. 1, FD&C Blue No. 2, FD&C Green No. 3, FD&C Red No. 40, FD&C Red No. 3, FD&C Yellow No. 5, and FD&C Red No. 6, caramel coloring, annatto, cochineal, betanin, turmeric, saffron, paprika, pandan, and butterfly pea. Non-limiting examples of fluorescent dyes include, but are not limited to, fluorescein sodium, rhodamine B, Acridine Orange, green fluorescent protein, curcumin, and zinc sulfide. In preferred embodiments, the dye does not affect the efficacy or bioavailability of the toxin in the topical toxin composition.

In other embodiments, the indicator may comprise inert, colored particles that are mixed into the topical toxin composition to allow for easier visual identification of the composition. For example, small particles of titanium dioxide may be added to impart a white color to the topical toxin composition. The use of titanium oxide as an indicator also has the added advantage that the titanium oxide particles may act as an inactivation agent in the presence of UV radiation, as discussed below. However, when titanium dioxide is used as an indicator, care must be taken to avoid exposing the topical toxin composition to UV light prematurely, in order to avoid inadvertently inactivating the toxin via photocatalytic degradation reactions.

Topical toxin composition is permitted to dwell on the skin for a period of time after application to allow the toxin to penetrate the skin. The duration of this dwell time may vary depending on the particular topical toxin composition, the amount of toxin to be delivered, and the area to be treated. Non-limiting examples of dwell times that may be used include, but is not limited to, 1 minute, 2 minutes, 3 minutes, 4 minutes, 5 minutes, 6 minutes, 7 minutes, 8 minutes, 9 minutes, 10 minutes, 15 minutes, 20 minutes, 25 minutes, 30 minutes, 35 minutes, 40 minutes, 45 minutes, 50 minutes, 55 minutes, 1 hour, 1.25 hours, 1.5, hours, 1.75 hours, or 2 hours.

This kit of the invention also comprises a removal agent for removing excess toxin from the treated area, said removal agent comprising an agent that absorbs, adsorbs, or physically scrapes off the excess toxin. The removal agent may also remove topical toxin compositions from surfaces whenever the presence of the topical toxin compositions on such surfaces is not desired. For instance, the removal agent may be used to remove excess topical toxin composition which has been applied to an area of skin to be treated. Alternatively, the removal agent may be used to remove topical toxin composition that has been inadvertently administered to a area of skin not in need of treatment. The invention also contemplates using the removal agent to remove toxin from the surface of any article that has been contaminated with toxin. For example, examples of articles in a clinical setting that may become contaminated by a topical toxin composition include tabletops, chairs, floors, benches, gloves, glasses, applicators, and the like.

Generally speaking, the removal agent is for removing excess toxin from the treated area of skin, said removal agent comprising an agent that absorbs, adsorbs, or physically scrapes off the excess toxin. In such embodiments, the removal agent may comprise any material/article capable of removing the composition including, but not limited to, synthetic and/or natural polymeric articles, metal articles, ceramic articles, and glass articles. Polymeric articles may comprise cellulose and/or derivatives of cellulose or plastics. Non-limiting examples of removal agents include, but are not limited to, spatulas, materials comprising cotton, materials comprising rayon, paper towels, gauze, swabs, cotton balls, surgical draping, facial tissue, disposable absorbent materials, disposable adsorbent materials, disposable cloths, disposable woven and non-woven fabrics, wet wipes, and dry wipes.

Removing the excess toxin may involve wiping, blotting, scraping or rubbing the surface area with the removal agent. The removal agent may also be placed directly on a surface for a period of time to allow the topical toxin composition to absorb into or adsorb onto the removal agent. Where the topical toxin composition comprises an indicator, the removal agent may be repeatedly administered by the clinician until the indicator is no longer visible or present in an amount that is below an acceptable upper limit, thereby indicating that most, if not all, of the topical toxin composition has been removed. For example, when the indicator is a dye, this threshold limit may be expressed as a percentage of the amount of dye originally present, as an absolute number, or in any other way known to one of skill in the art. Threshold limits of dye contemplated by the invention may be any known to one of skill in the art including, but not limited to, less than 20% of that originally present, less than 10% of that originally present, less than 5% of that originally present, less than 1% of that originally present, less than 0.5% of that originally present, less than 0.1% of that originally present, less than 0.05% of that originally present and 0.01% of that originally present. This invention also contemplates the use of more than one and/or more than one type of removal agent to remove undesired toxin from a surface.

The kit of the invention comprises the removal agent described herein and an inactivation agent. The inactivation agent is any agent that inactivates excess toxin, thereby rendering the toxin without neurotoxicity. Non-limiting examples of inactivation agents include oxidizers, surfactants, oxidizers, electromagnetic radiation, and basic agents.

The removal agent and the inactivation agent are used concurrently. For example, in one particularly preferred example, a wipe that has been impregnated with hypochlorite bleach may be used to physically remove topical toxin on a surface while inactivating it at the same time. The removal agent and the inactivation agent are used sequentially. For example, after the removal agent physically removes toxin from a surface, the removal agent may be exposed to an inactivation agent to render harmless the toxin in contact with the removal agent. Alternatively, the removal agent and the inactivation agent may be sequentially applied to a surface to decontaminate the surface from topical toxin present thereon.

One aspect of the invention is the recognition that certain classes of surfactants inactivate botulinum toxin, while other classes of surfactants do not. Generally speaking, strong ionic detergents are useful as inactivation agents, non-limiting examples of which include sodium dodecyl sulfate, sodium lauroyl lactylate, sodium laureth sulfate, and sodium lauroyl sarcosinate. For example, contact of botulinum toxin type A with Dove Facial Cleaning Cloths, Deep Moisture^{®} was found to inactivate botulinum toxin type A. These cleaning cloths list the following substances as ingredients: water, glycerin, decyl glucoside, sodium lauroyl sarcosinate, cocamidopropyl betaine, silicone quaternium-8, sodium lauroyl lactylate, polyquaternium-10, tocopheryl acetate (vitamin E acetate), retinyl palmitate (vitamin A palmitate), potassium lactate, lactic acid, cholesterol glycine soja (soybean) sterol, urea sodium Pca, polyquaternium-4, fragrance/parfum, Dmdm hydantoin, and iodopropynyl butylcarbamate. Thus, while strong ionic detergents, such as those listed above, are useful for inactivating botulinum toxin, they are often inappropriate for cleansing the treatment area either before or after administration of botulinum toxin, since they destroy the activity of the botulinum toxin.

By contrast, non-ionic surfactants (e.g., polysorbate 20, polysorbate 40, polysorbate 60, polysorbate 80, Span 20, Span 60, Span 65, Span 80, PEG 4 Laurate, and PEG 6 Caprylic/Capric Glycerides) generally are less useful as inactivation agents, particularly at low concentrations, but may be used advantageously to cleanse an area of skin prior to treatment or as a part of a removal agent to enhance the removal of topical botulinum toxin from a treated area of skin. For example, Neutrogena Makeup Remover Cleansing Towelettes^{®}, which contain non-ionic surfactants, can be used as a post-treatment wipe to clean off excess botulinum toxin on an area of skin after adminstration of the topical botulinum toxin composition. Neutrogena Makeup Remover Cleansing Towelettes^{®} contain the following ingredients: water, cetyl ethylhexanoate, isostearyl palmitate, pentaerythrityl tetraethylhexanoate, isononyl isononanoate, cyclopentasiloxane hexylene glycol, PEG 4 laurate , PEG 6 caprylic/capric glycerides, sucrose cocoate, carbomer, sodium hydroxide, benzoic acid, dehydroacetate acid, phenoxyethanol, iodopropynyl butylcarbamate, and fragrance. In addition, Neutrogena Makeup Remover Cleansing Towelettes^{®} can be used as a pre-treatment wipe to cleanse the area to be treated, without deleteriously affecting the activity of the subsequently applied topical botulinum toxin composition. In embodiments involving non-ionic surfactants that do not inactivate botulinum toxin, an inactivation agent is preferably used to neutralize excess botulinum toxin. For example, in certain preferred embodiments, the inactivation agent may be solid or solution forms of sodium hypochlorite (e.g., bleach) as described herein. In addition, this invention also contemplates the use of other chemical substances as a removal agent or a part of a removal agent. For example, excess botulinum toxin type A on the surface of a patient's skin may be removed, without significant inactivation, by wiping with a gauze pad moistened with 70% isopropyl alcohol.

In certain preferred embodiments, the inactivation agent is an oxidizer. For example, suitable oxidizers include any chemical substance capable of oxidizing the toxin to render it harmless. Non-limiting examples of suitable oxidizers include ozone gas, ozonated water, peroxides, and hypochlorites. In certain preferred embodiments, the oxidizer is a hypochlorite salts, which may be used alone or in combination with other inactivation agents. Suitable hypochlorite salts include, but are not limited to, sodium hypochlorite and calcium hypochlorite. The hypochlorite salt may be in any form and/or concentration known to be useful for inactivating the composition. Non-limiting examples of forms of hypochlorite salts include, liquid, powder, crystalline salt, and mist. In some instances, sodium hypochlorite used as an inactivation agent is referred to as bleach. The concentration of hypochlorite salt may be, but is not limited to, at least 0.1%, approximately 0.1% to 50%, 0.1% to 12%, 0.3% to 10%, or 0.5% to 10%. In preferred embodiments, the hypochlorite salt is sodium hypochlorite at a concentration between approximately 0.1% to 6%.

Electromagnetic radiation may be used as an inactivation agent, alone or in combination with other inactivation agents. The electromagnetic radiation suitable for use as an inactivation agent includes any radiation of any wavelength that is capable of degrading the toxin so as to render in non-toxic. In certain preferred embodiments, the inactivation agent may include, but is not limited to, gamma rays, x-rays, and ultraviolet radiation. The electromagnetic radiation may be generated naturally or artificially. In preferred embodiments, the electromagnetic radiation includes wavelengths between approximately 10 nm to 400 nm. In instances where the electromagnetic radiation is ultraviolet radiation (UV), any source of UV known to one of skill in the art may be used. This may include, but is not limited to, solar-related sources, "black lights" or Wood's light, UV fluorescent lamps, UV light-emitting diodes (UV LEDs), UV lasers, and gas-discharge lamps. In certain embodiments, inactivation agents comprising electromagnetic radiation further comprise titanium dioxide. While not intending to be bound to any theory, titanium dioxide may act as a photocatalyst to improve the inactivation agent's ability to degrade the composition.

Agents that alter the pH may be used as inactivation agents, alone or in combination with other inactivation agents. Any substance that alters the pH to a value that causes degradation of the toxin may be used as a pH-altering inactivation agent. Non-limiting examples pH-altering inactivation agents suitable for the invention include, potassium hydroxide (KOH), barium hydroxide (Ba(OH)₂), caesium hydroxide (CsOH), sodium hydroxide (NaOH), strontium hydroxide (Sr(OH)₂), calcium hydroxide (Ca(OH)₂), lithium hydroxide (LiOH), and rubidium hydroxide (RbOH).

Heat may be used as an inactivation agent, alone or in combination with other inactivation agents. In instances where the composition is a toxin and particularly a botulinum toxin, heating the composition to temperatures greater than 85 °C for at least 5 minutes will inactivate the toxin. In certain preferred embodiments, the toxin is inactivated by heating it to greater than 70 °C, approximately 70 to 100 °C, 70 to 80 °C , 80 to 90 °C, 85 to 90°C, 86 to 90 °C , 87 to 90°C, 88 to 90 °C, 89 to 90°C, 86 °C , 87 °C, 88 °C , 89 °C, 90 °C, 90 to 100 °C, 100 to 110 °C, 110 to 120 °C, 120 to 130 °C, greater than 130 °C but less than a temperature that would damage the article being decontaminated. The heat source used may be any of those known to one of skill in the art including, but not limited to, resistive heating elements, laboratory heat blocks, heated water baths, autoclaves, ovens, and microwave ovens.

In certain embodiments, antitoxin may be used as an inactivation agent, alone or in combination with other inactivation agents. The antitoxin may be derived from any source known to one of skill in the art including, but not limited to, equine, murine, porcine, canine, human, and the like. The antitoxin may comprise whole antibodies, partial antibodies, and/or non-antibody components. In certain preferred embodiments, the antitoxin is equine trivalent botulinum antitoxin. In other preferred embodiments, the antitoxin is equine heptavalent botulinum antitoxin.

The inactivation agent may degrade the composition by any means possible including, but not limited to, via thermal, chemical, photochemical, and pH fluctuating processes. Any method known to one of skill in the art to inactive the composition may be used.

Kits for applying topical therapeutic or cosmetic compositions are also encompassed by this invention. The kit comprises a composition for topical application to an area in need of treatment. The kit also comprises an indicator to allow for monitoring the location and presence of the composition, further wherein the indicator is for indicating the area of skin that has been treated by the composition. The kit includes a composition comprising botulinum toxin, and optionally, a detectable dye. In preferred embodiments, the botulinum toxin is purified botulinum toxin type A. The kit further comprises an applicator for topically applying the composition to an area in need of treatment. The applicator may comprise any component required for administration of the composition, including a custom applicator, a glove, or a syringe. The kit also includes a removal agent for removing excess toxin from the treated area, said removal agent comprising an agent that absorbs, adsorbs, or physically scrapes off the excess toxin. In preferred embodiments, the removal agent is a cellulose-based polymeric article, for example, a paper towel, gauze, a swab, or a cotton ball. The kit further comprises an inactivation agent that is used to inactivate the excess composition upon removal from the treated area, thereby rendering the toxin without neurotoxicity. The inactivation agent may comprise any component known to inactivate the removed composition. In certain embodiments, the inactivation agent includes surfactants, sodium hypochlorite, ultraviolet radiation, acidic or basic agents, and resistive heating elements. The kit may also, in certain embodiments, include instructions for use of the components contained therein.

The specific embodiments described herein are offered by way of example only, and the invention is to be limited only by the terms of the appended claims, along with the full scope of the equivalents to which such claims are entitled.

### EXAMPLES

### Example 1: Topical Toxin Compositions Suitable for Use with the Methods and Kits of Removing and Inactivating Toxin

As described herein, the methods and kits for removing and inactivating topical toxin compositions are not particularly limited. Generally, they may be used with any delivery system in which toxin is applied topically to the skin. For example, the methods and kits are suitable for use with transdermal patches that deliver toxin to the skin. The delivery system may involve manually applying the topical toxin formulation directly onto the area to be treated. When the topical toxin composition is manually applied in this manner, it may be spread about using a custom applicator or with by hand, provided that the hand is protected by a glove made of a suitable material (e.g., nitrile).

In general, the methods and kits described herein are suitable for removing and inactivating the toxin in a wide variety of different topical toxin compositions. For instance, the methods and kits are suitable for use with topical toxin compositions in which the toxin is delivered by means of a carrier or a skin-penetration enhancer. Non-limiting examples of carriers include positively charged polymeric carriers and micelles (e.g., phospholipid micelles such as sphingosine and cerebroside). Non-limiting examples of skin-penetration enhancers include DMSO, d-limonene, allantoin, fulvic acid, myrrh, hydroquinone glyquin, quillaja saponaria, and acanthophyllum squarrusom.

The topical compositions used with the methods and kits may comprise a positively charged carrier that promotes transdermal transport of the toxin. See, *e.g.,* U.S. Publication No. 2005/0196414 entitled "Compositions and Methods for Topical Application and Transdermal Delivery of Botulinum Toxin". For example, the carrier may be a positively charged carrier molecule with positively charged efficiency groups attached thereto. In certain preferred embodiments, the topical transport is enhanced by the carrier without covalent modification of the therapeutic or cosmetic active agent to be delivered.

By "positively charged" is meant that the carrier has a positive charge under at least some solution-phase conditions, more preferably under at least some physiologically compatible conditions. Generally, the positively-charged carrier comprises a "positively charged backbone," which is typically a linear chain of atoms, either with groups in the chain carrying a positive charge at physiological pH, or with groups carrying a positive charge attached to side chains extending from the backbone. Preferably, the positively charged backbone itself will not have a defined enzymatic or therapeutic biologic activity. The linear backbone is a hydrocarbon backbone which is, in some embodiments, interrupted by heteroatoms selected from nitrogen, oxygen, sulfur, silicon and phosphorus. The majority of backbone chain atoms are usually carbon. Additionally, the backbone will often be a polymer of repeating units (e.g., amino acids, poly(ethyleneoxy), poly(propyleneamine), polyalkyleneimine, and the like) but can be a heteropolymer. In one group of embodiments, the positively charged backbone is a polypropyleneamine wherein a number of the amine nitrogen atoms are present as ammonium groups (tetra-substituted) carrying a positive charge. In another embodiment, the positively charged backbone is a nonpeptidyl polymer, which may be a hetero- or homo-polymer such as a polyalkyleneimine, for example a polyethyleneimine or polypropyleneimine, having a molecular weight of from about 10,000 to about 2,500,000, preferably from about 100,000 to about 1,800,000, and most preferably from about 500,000 to about 1,400,000. In another group of embodiments, the backbone has attached a plurality of side-chain moieties that include positively charged groups (*e.g.,* ammonium groups, pyridinium groups, phosphonium groups, sulfonium groups, guanidinium groups, or amidinium groups). The sidechain moieties in this group of embodiments can be placed at spacings along the backbone that are consistent in separations or variable. Additionally, the length of the sidechains can be similar or dissimilar. For example, in one group of embodiments, the sidechains can be linear or branched hydrocarbon chains having from one to twenty carbon atoms and terminating at the distal end (away from the backbone) in one of the above-noted positively charged groups. In all aspects of the present invention, the association between the carrier and the therapeutic or cosmetic active agent is by noncovalent interaction, non-limiting examples of which include ionic interactions, hydrogen bonding, van der Waals forces, or combinations thereof.

In one group of embodiments, the positively charged backbone is a polypeptide having multiple positively charged sidechain groups (e.g., lysine, arginine, ornithine, homoarginine, and the like). Preferably, the polypeptide has a molecular weight of from about 10,000 to about 1,500,000, more preferably from about 25,000 to about 1,200,000, most preferably from about 100,000 to about 1,000,000. One of skill in the art will appreciate that when amino acids are used in this portion of the invention, the sidechains can have either the D- or L-form (R or S configuration) at the center of attachment. Alternatively, the backbone can be an analog of a polypeptide such as a peptoid. See, for example, Kessler, Angew. Chem. Int. Ed. Engl. 32:543 (1993); Zuckermann et al. Chemtracts-Macromol. Chem. 4:80 (1992); and Simon et al. Proc. Nat'l. Acad. Sci. USA 89:9367 (1992)). Briefly, a peptoid is a polyglycine in which the sidechain is attached to the backbone nitrogen atoms rather than the α-carbon atoms. As above, a portion of the sidechains will typically terminate in a positively charged group to provide a positively charged backbone component. Synthesis of peptoids is described in, for example, U.S. Pat. No. 5,877,278. As the term is used herein, positively charged backbones that have a peptoid backbone construction are considered "non-peptide" as they are not composed of amino acids having naturally occurring sidechains at the α-carbon locations.

A variety of other backbones can be used employing, for example, steric or electronic mimics of polypeptides wherein the amide linkages of the peptide are replaced with surrogates such as ester linkages, thioamides (--CSNH--), reversed thioamide (--NHCS-), aminomethylene (--NHCH₂--) or the reversed methyleneamino (--CH₂NH--) groups, ketomethylene (--COCH₂--) groups, phosphinate (--PO₂RCH₂--), phosphonamidate and phosphonamidate ester (--PO₂RNH--), reverse peptide (--NHCO--), trans-alkene (--CR=CH-), fluoroalkene (--CF=CH--), dimethylene (--CH₂CH₂--), thioether (--CH₂S--), hydroxyethylene (--CH(OH)CH₂--), methyleneoxy (--CH₂O--), tetrazole (CN₄), sulfonamido (--SO₂NH--), methylenesulfonamido (--CHRSO₂NH--), reversed sulfonamide (--NHSO₂--), and backbones with malonate and/or gem-diamino-alkyl subunits, for example, as reviewed by Fletcher et al. ((1998) Chem. Rev. 98:763) and detailed by references cited therein. Many of the foregoing substitutions result in approximately isosteric polymer backbones relative to backbones formed from α-amino acids.

In each of the backbones provided above, sidechain groups can be appended that carry a positively charged group. For example, the sulfonamide-linked backbones (--SO₂NH-- and --NHSO₂--) can have sidechain groups attached to the nitrogen atoms. Similarly, the hydroxyethylene (--CH(OH)CH₂--) linkage can bear a sidechain group attached to the hydroxy substituent. One of skill in the art can readily adapt the other linkage chemistries to provide positively charged sidechain groups using standard synthetic methods.

In one embodiment, the positively charged backbone is a polypeptide having efficiency groups. As used herein, an efficiency group is any agent that has the effect of promoting the translocation of the positively charged backbone through a tissue or cell membrane. Non-limiting examples of efficiency groups include -(gly)ₙ₁-(arg)ₙ₂, HIV-TAT or fragments thereof, or the protein transduction domain of Antennapedia, or a fragment thereof, in which the subscript n1 is an integer of from 0 to 20, more preferably 0 to 8, still more preferably 2 to 5, and the subscript n2 is independently an odd integer of from about 5 to about 25, more preferably about 7 to about 17, most preferably about 7 to about 13. Still further preferred are those embodiments in which the HIV-TAT fragment has the formula (gly)ₚ-RGRDDRRQRRR-(gly)_{q}, (gly)ₚ-YGRKKRRQRRR-(gly)_{q} or (gly)ₚ-RKKRRQRRR-(gly)_{q} wherein the subscripts p and q are each independently an integer of from 0 to 20 and the fragment is attached to the backbone via either the C-terminus or the N-terminus of the fragment. Preferred HIV-TAT fragments are those in which the subscripts p and q are each independently integers of from 0 to 8, more preferably 2 to 5. In some embodiments, the carrier has the amino acid sequence RKKRRQRRR-G-(K)₁₅-G-RKKRRQRRR.

In another preferred embodiment the positively charged efficiency group is the Antennapedia (Antp) protein transduction domain (PTD), or a fragment thereof that retains activity. (See, *e.g.,* Console et al., J. Biol. Chem. 278:35109 (2003)). Preferably the positively charged carrier includes side-chain positively charged efficiency groups in an amount of at least about 0.05%, as a percentage of the total carrier weight, preferably from about 0.05 to about 45 weight %, and most preferably from about 0.1 to about 30 weight %. For positively charged efficiency groups having the formula -(gly)ₙ₁-(arg)ₙ₂, the most preferred amount is from about 0.1 to about 25%.

In another embodiment, the backbone portion is a polylysine and positively charged efficiency groups are attached to the lysine sidechain amino groups. In some embodiments, the polylysine may have a molecular weight that ranges from about 10,000 to about 1,500,000, preferably from about 25,000 to about 1,200,000, and most preferably from about 100,000 to about 1,000,000. In other embodiments, the polylysine may have a molecular weight that ranges from about 500 to about 5000. about 1000 to about 4000, about 1500 to about 3500, or about 2000 to about 3000. The polylysine may be any of the commercially available (Sigma Chemical Company, St. Louis, Mo., USA) polylysines such as, for example, polylysine having MW>70,000, polylysine having MW of 70,000 to 150,000, polylysine having MW 150,000 to 300,000 and polylysine having MW>300,000. The selection of an appropriate polylysine will depend on the remaining components of the composition and will be sufficient to provide an overall net positive charge to the composition and, in some embodiments, provide a length that is preferably from one to four times the combined length of the negatively charged components. Preferred positively charged efficiency groups or efficiency groups include, for example, -gly-gly-gly-arg-arg-arg-arg-arg-arg-arg (-Gly₃Arg₇) or HIV-TAT. In another preferred embodiment the positively charged backbone is a long chain polyalkyleneimine such as a polyethyleneimine, for example, one having a molecular weight of about 1,000,000.

In another embodiment, the carrier is a polylysine with positively charged branching groups attached to the lysine side-chain amino groups. The polylysine used in this particularly embodiment can be any of the commercially available (Sigma Chemical Company, St. Louis, Mo., USA, e.g.) polylysines such as, for example, polylysine having MW>70,000, polylysine having MW of 70,000 to 150,000, polylysine having MW 150,000 to 300,000 and polylysine having MW>300,000. However, preferably the polylysine has MW of at least about 10,000. Preferred positively charged branching groups or efficiency groups include, for example, -gly-gly-gly-arg-arg-arg-arg-arg-arg-arg (-Gly₃Arg₇), HIV-TAT or fragments of it, and Antennapedia PTD or fragments thereof.

In other embodiments of this invention, the carrier is a relatively short polylysine or polyethyleneimine (PEI) backbone (which may be linear or branched) and which has positively charged branching groups. Such carriers are useful for minimizing uncontrolled aggregation of the backbones and botulinum toxin in a therapeutic composition, which causes the transport efficiency to decrease dramatically. When the carrier is a relatively short linear polylysine or PEI backbone, the backbone will have a molecular weight of less than 75,000, more preferably less than 30,000, and most preferably, less than 25,000. When the carrier is a relatively short branched polylysine or PEI backbone, however, the backbone will have a molecular weight less than 60,000, more preferably less than 55,000, and most preferably less than 50,000.

### Example 2: Application, Removal and Inactivation of Botulinum Toxin A From Skin.

A person with undesired glabellar lines visits a clinician to have them treated. The clinician initially cleanses the area to be treated by wiping it with a cotton ball that has been moistened with deionized water. The clinician then prepares a topical toxin composition by reconstituting a lyophilized formulation containing isolated botulinum toxin type A neurotoxin, and a positively charged carrier as described in Example 2. The clinician applies the topical toxin composition onto the intended treatment area and then spreads it using her nitrile-gloved finger.

The clinician then wipes the contaminated glove with a moist wipe that has been impregnated by sodium hypochlorite (i.e., a bleach wipe). The bleach wipe physically removes the topical toxin composition and chemically inactivates the botulinum toxin. The clinician then places the used bleach wipe in a hermetic container containing sodium hypochlorite liquid to help ensure that any residual botulinum toxin is inactivated.

After the topical toxin composition is given time to penetrate the treated area of skin, the clinician removes the excess topical toxin composition by blotting the treated areas with a paper towel. The clinician then places the used paper towel and nitrile gloves in the hermetic container for safe disposal.

### Example 3: Application, Removal and Inactivation of a Topical Botulinum Composition Containing an Indicator

A person suffering from hyperhidrosis visits a doctor for treatment. The doctor initially cleanses the underarm area of the patient by wiping it with cotton balls that have been moistened with deionized water. Prior to treatment, the doctor dries the area to be treated. The doctor then prepares a topical toxin composition by reconstituting a lyophilized formulation containing isolated botulinum toxin type A neurotoxin, a positively charged carrier as described in Example 2, and an indicator comprising fine titanium dioxide particles. The doctor applies the topical toxin composition onto the intended treatment area, and then spreads it by rubbing her nitrile-gloved finger on the area to be treated. The presence of the titanium dioxide in the reconstituted formulation renders the formulation white, allowing the doctor to see more easily which areas of the underarm skin have already been treated.

After the topical toxin composition is given time to penetrate the treated area of skin, the doctor removes the excess topical toxin composition by blotting the treated areas with a paper towel. The doctor then places the contaminated paper towel and nitrile gloves in a decontamination chamber that is equipped with a UV light source. By turning on the UV light source, the doctor initiates photocatalytic reactions involving the titanium dioxide particles that degrade the botulinum toxin, thereby rendering the toxin harmless.

Between patients, the doctor turns on a UV light in the treatment room, in order to photocatalytically degrade any toxin that may have been inadvertently applied to a surface in the treatment room.

### Example 4: Application, Removal and Inactivation of a Topical Botulinum Composition Using a Surfactant

A person with undesired glabellar lines visits a clinician to have them treated. The clinician initially cleanses the area to be treated by wiping it with a cotton ball that is moistened with deionized water. The clinician then prepares a topical toxin composition by reconstituting a lyophilized formulation containing isolated botulinum toxin type A neurotoxin, and a positively charged carrier as described in Example 2. The clinician applies the topical toxin composition onto the intended treatment area, and then spreads it with her nitrile-gloved finger.

The clinician then wipes the contaminated glove with a moist wipe that has been impregnated by sodium hypochlorite (i.e., a bleach wipe). The bleach wipe physically removes the topical toxin composition and chemically inactivates the botulinum toxin. The clinician then places the used bleach wipe in a hermetic container containing sodium hypochlorite liquid to help ensure that an residual botulinum toxin is inactivated.

After the topical toxin composition is given time to penetrate the treated area of skin, the clinician removes the excess topical toxin composition by blotting the treated areas with a paper towel. The clinician then swabs the treated areas with a cotton swab that has been previously dipped in a surfactant. The surfactant removes the excess toxin at the surface of the treated areas, without affecting the toxin that has already penetrated the skin.

The clinician then places the used paper towel, swab, and nitrile gloves in the hermetic container for safe disposal.

### Example 5: Application, Removal, and Inactivation of a Topical Botulinum Toxin Composition for Treating Lateral Canthal Lines

A patient with undesired lateral canthal lines was treated with a topical botulinum toxin composition. Prior to administration of a topical botulinum toxin composition, the lateral canthal areas were cleansed with gauze pads moistened with water. The topical botulinum toxin composition, which comprised purified botulinum toxin molecules and a positively charged backbone carrier with attached HIV-TAT efficiency groups, was applied in droplets on one of the lateral canthal areas of the patient using a syringe with a blunt needle. The topical botulinum toxin composition was massaged into the lateral canthal area using a gloved finger. Topical botulinum toxin composition was applied in a similar manner to the other lateral canthal area of the patient. Subsequently, both lateral canthal areas were covered with an occlusive dressing. The subject was allowed to rest for a rest period of 30 ±5 minutes. After the rest period, the lateral canthal areas were gently swabbed with a series of gauze pads moistened with water.

The used materials (e.g., syringes, occlusive material, gloves, and gauze) were disposed of in a biohazard bag. To inactivate the botulinum toxin, the used materials were heated at about 120 °C for about 60 minutes. A noticeable reduction in the appearance of lateral canthal lines was observed after 5 days. After two weeks, a substantial reduction in the appearance of lateral canthal lines was observed. The greatest reduction in the appearance of lateral canthal lines (i.e., the peak effect) occurred at about 28 days after administration of the topical botulinum toxin composition.

## Claims

1. A kit for topically applying a botulinum toxin for transdermal delivery, the kit comprising:
a composition comprising botulinum toxin and an indicator to allow for monitoring the location and presence of the composition, further wherein the indicator is for indicating the area of skin that has been treated by the composition;
an applicator for topically applying the composition to an area in need of treatment;
a removal agent for removing excess toxin from the treated area, said removal agent comprising an agent that absorbs, adsorbs, or physically scrapes off the excess toxin; and
an inactivation agent for inactivating the excess toxin, thereby rendering the toxin without neurotoxicity.

2. The kit according to claim 1, wherein the botulinum toxin is botulinum toxin serotype A.

3. The kit according to claim 2, wherein the botulinum toxin is an isolated botulinum toxin molecule.

4. The kit according to claim 3, wherein the isolated botulinum toxin does not comprise stabilizing proteins expressed by C. *botulinum.*

5. The kit according to any one of claims 1 to 4, wherein the indicator is a detectable dye.

6. The kit according to any one of claims 1 to 5, wherein the carrier comprises a positively charged polypeptide backbone having attached thereto one or more efficiency groups selected from the group consisting of (gly)ₚ-RGRDDRRQRRR-(gly)_{q}, (gly)ₚ-YGRKKRRQRRR-(gly)_{q}, and (gly)ₚ-RKKRRQRRR(gly)_{q}, wherein the subscripts p and q are each independently an integer of from 0 to 20, and said efficiency group is attached to said backbone via either the C- or N- terminus of the efficiency group.

7. The kit according to claim 6, wherein the carrier has the amino acid sequence RKKRRQRRR-G-(K)₁₅-G-RKKRRQRRR.

8. The kit according to any one of claims 1 to 7, wherein the applicator comprises a glove or a syringe.

9. The kit according to any one of claims 1 to 8, wherein the removal agent comprises a cellulose-based polymeric article.

10. The kit according to claim 9, wherein the polymeric article is selected from the group consisting of a paper towel, a cotton ball, gauze, and a swab.

11. The kit according to any one of claims 1 to 10, wherein the inactivation agent comprises an oxidizer.

12. The kit according to claim 11, wherein the oxidizer is biodegradable.

13. The kit according to any one of claims 1 to 12, wherein the inactivation agent comprises sodium hypochlorite salt.

14. The kit according to claim 13, wherein the sodium hypochlorite salt is in a form selected from the group consisting of a liquid, a powder, a crystalline salt, and a mist.

15. The kit according to any one of claims 1 to 11, wherein the inactivation agent comprises a source of electromagnetic radiation for photochemically degrading the toxin.

16. The kit according to claim 15, wherein the electromagnetic radiation comprises ultraviolet radiation.

17. The kit according to any one of claims 1 to 16, wherein the composition comprising said botulinum toxin for transdermal delivery further comprises titanium dioxide.

18. The kit according to any one of claims 5 to 17, wherein the dye is at least one selected from the group consisting of an artificial dye, a natural food dye, FD&C Blue No. 1, FD&C Blue No. 2, FD&C Green No. 3, FD&C Red No. 40, FD&C Red No. 3, FD&C Yellow No. 5, FD&C Red No. 6, caramel coloring, annatto, cochineal, betanin, turmeric, saffron, paprika, pandan, and butterfly pea.

19. The kit according to any one of the preceding claims, wherein the botulinum toxin is present in the composition to provide a dose of botulinum toxin from 100 U to 7000 U.

20. The kit according to claim 19, wherein the botulinum toxin is present in the composition to provide a dose of botulinum toxin from 500 U to 6000 U.

21. The kit according to claim 20, wherein the botulinum toxin is present in the composition to provide a dose of botulinum toxin from 1000 U to 5000 U.

22. The kit according to claim 21, wherein the botulinum toxin is present in the composition to provide a dose of botulinum toxin from 2000 U to 4000 U.

## Patentansprüche

1. Kit zum topischen Anwenden eines Botulinumtoxins für die transdermale Abgabe, wobei der Kit umfasst:
eine Zusammensetzung, die Botulinumtoxin und einen Indikator umfasst, um das Überwachen des Ortes und des Vorhandenseins der Zusammensetzung zu ermöglichen, wobei der Indikator ferner dazu dient, den Bereich der Haut anzuzeigen, der mit der Zusammensetzung behandelt wurde;
einen Applikator zum topischen Anwenden der Zusammensetzung auf einen behandlungsbedürftigen Bereich;
ein Entfernungsmittel zum Entfernen von überschüssigem Toxin von dem behandelten Bereich, wobei das Entfernungsmittel ein Mittel umfasst, das das überschüssige Toxin absorbiert, adsorbiert oder physisch abschabt; und
ein Inaktivierungsmittel zum Inaktivieren des überschüssigen Toxins, wodurch dem Toxin seine Neurotoxizität genommen wird.

2. Kit nach Anspruch 1, wobei das Botulinumtoxin Botulinumtoxin des Serotyps A ist

3. Kit nach Anspruch 2, wobei das Botulinumtoxin ein isoliertes Botulinumtoxinmolekül ist.

4. Kit nach Anspruch 3, wobei das isolierte Botulinumtoxin keine von C. *botulinum* exprimierten stabilisierenden Proteine umfasst.

5. Kit nach einem der Ansprüche 1 bis 4, wobei der Indikator ein nachweisbarer Farbstoff ist.

6. Kit nach einem der Ansprüche 1 bis 5, wobei der Träger ein positiv geladenes Polypeptidrückgrat umfasst, das eine oder mehrere daran angelagerte Effizienzgruppen aufweist, die aus der Gruppe ausgewählt sind, bestehend aus (gly)ₚ-RGRDDRRQRRR-(gly)_{q}, (gly)ₚ-YGRKKRRQRRR-(gly)_{q} und (gly)ₚ-RKKRRQRRR(gly)_{q}, wobei die Indizes p und q jeweils unabhängig voneinander eine ganze Zahl von 0 bis 20 sind und die Effizienzgruppe entweder über den C- oder N-Terminus der Effizienzgruppe an das Rückgrat angelagert ist.

7. Kit nach Anspruch 6, wobei der Träger die Aminosäuresequenz RKKRRQRRR-G-(K)₁₅-G-RKKRRQRRR aufweist.

8. Kit nach einem der Ansprüche 1 bis 7, wobei der Applikator einen Handschuh oder eine Spritze umfasst

9. Kit nach einem der Ansprüche 1 bis 8, wobei das Entfernungsmittel einen Polymerartikel auf Cellulosebasis umfasst.

10. Kit nach Anspruch 9, wobei der Polymerartikel aus der Gruppe ausgewählt ist, bestehend aus einem Papierhandtuch, einem Wattebausch, Verbandmull und einem Tupfer

11. Kit nach einem der Ansprüche 1 bis 10, wobei das Inaktivierungsmittel ein Oxidationsmittel umfasst.

12. Kit nach Anspruch 11, wobei das Oxidationsmittel biologisch abbaubar ist.

13. Kit nach einem der Ansprüche 1 bis 12, wobei das Inaktivierungsmittel Natriumhypochloritsalz umfasst.

14. Kit nach Anspruch 13, wobei das Natriumhypochloritsalz in einer Form vorliegt, die aus der Gruppe ausgewählt ist, bestehend aus einer Flüssigkeit, einem Pulver, einem kristallinen Salz und einem Nebel.

15. Kit nach einem der Ansprüche 1 bis 11, wobei das Inaktivierungsmittel eine Quelle elektromagnetischer Strahlung zum photochemischen Abbauen des Toxins umfasst.

16. Kit nach Anspruch 15, wobei die elektromagnetische Strahlung ultraviolette Strahlung umfasst.

17. Kit nach einem der Ansprüche 1 bis 16, wobei die Zusammensetzung, die das Botulinumtoxin für die transdermale Abgabe umfasst, ferner Titandioxid umfasst.

18. Kit nach einem der Ansprüche 5 bis 17, wobei der Farbstoff wenigstens einer ist, der aus Gruppe ausgewählt ist, die aus einem künstlichen Farbstoff, einem natürlichen Lebensmittelfarbstoff, FD&C Blue No. 1, FD&C Blue No. 2, FD&C Green No. 3, FD&C Red No. 40, FD&C Red No. 3, FD&C Yellow No. 5, FD&C Red No. 6, Karamellfarbstoff, Annatto, Cochenille, Betanin, Kurkuma, Safran, Paprika, Pandanus und Schmetterlingserbse besteht.

19. Kit nach einem der vorhergehenden Ansprüche, wobei das Botulinumtoxin in der Zusammensetzung vorhanden ist, um eine Dosis von 100 U bis 7000 U Botulinumtoxin bereitzustellen.

20. Kit nach einem Anspruch 19, wobei das Botulinumtoxin in der Zusammensetzung vorhanden ist, um eine Dosis von 500 U bis 6000 U Botulinumtoxin bereitzustellen.

21. Kit nach einem Anspruch 20, wobei das Botulinumtoxin in der Zusammensetzung vorhanden ist, um eine Dosis von 1000 U bis 5000 U Botulinumtoxin bereitzustellen.

22. Kit nach einem Anspruch 21, wobei das Botulinumtoxin in der Zusammensetzung vorhanden ist, um eine Dosis von 2000 U bis 4000 U Botulinumtoxin bereitzustellen.

## Revendications

1. Kit pour l'application topique d'une toxine botulique pour l'administration transdermique, le kit comprenant :
une composition comprenant une toxine botulique et un indicateur pour permettre la surveillance de l'emplacement et de la présence de la composition, en outre dans lequel l'indicateur est destiné à indiquer la zone de peau qui a été traitée par la composition ;
un applicateur destiné à appliquer topiquement la composition sur une zone ayant besoin de traitement ;
un agent d'élimination destiné à éliminer un excès de toxine de la zone traitée, ledit agent d'élimination comprenant un agent qui absorbe, adsorbe, ou enlève en grattant l'excès de toxine ; et
un agent d'inactivation destiné à inactiver l'excès de toxine, rendant ainsi la toxine non neurotoxique.

2. Kit selon la revendication 1, dans lequel la toxine botulique est la toxine botulique de sérotype A

3. Kit selon la revendication 2, dans lequel la toxine botulique est une molécule de toxine botulique isolée.

4. Kit selon la revendication 3, dans lequel la toxine botulique isolée ne comprend pas de protéine stabilisante exprimée par C. *botulinum.*

5. Kit selon l'une quelconque des revendications 1 à 4, dans lequel l'indicateur est un colorant détectable.

6. Kit selon l'une quelconque des revendications 1 à 5, dans lequel le vecteur comprend une chaîne principale polypeptidique de charge positive ayant, attachés à celle-ci, un ou plusieurs groupes efficaces sélectionnés parmi le groupe constitué de : (gly)ₚ-RGRDDRRQRRR-(gly)_{q}, (gly)ₚ-YGRKKRRQRRR-(gly)_{q}, et (gly)ₚ-RKKRRQRRR(gly)_{q}, dans lequel les indices inférieurs p et q sont chacun indépendamment un nombre entier de 0 à 20, et ledit groupe efficace est attaché à ladite chaîne principale par l'intermédiaire de l'un ou de l'autre du C- ou du N-terminus du groupe efficace.

7. Kit selon la revendication 6, dans lequel le vecteur a la séquence d'acides aminés RKKRRQRRR-G-(K)₁₅-G-RKKRRQRRR.

8. Kit selon l'une quelconque des revendications 1 à 7, dans lequel l'applicateur comprend un gant ou une seringue.

9. Kit selon l'une quelconque des revendications 1 à 8, dans lequel l'agent d'élimination comprend un article polymère à base de cellulose.

10. Kit selon la revendication 9, dans lequel l'article polymère est sélectionné parmi le groupe constitué de : une serviette en papier, un tampon de coton, une gaze, et un coton-tige.

11. Kit selon l'une quelconque des revendications 1 à 10, dans lequel l'agent d'inactivation comprend un oxydant.

12. Kit selon la revendication 11, dans lequel l'oxydant est biodégradable.

13. Kit selon l'une quelconque des revendications 1 à 12, dans lequel l'agent d'inactivation comprend du sel hypochlorite de sodium.

14. Kit selon la revendication 13, dans lequel le sel hypochlorite de sodium est sous une forme sélectionnée parmi le groupe constitué de : un liquide, une poudre, un sel cristallin, et de fines gouttelettes.

15. Kit selon l'une quelconque des revendications 1 à 11, dans lequel l'agent d'inactivation comprend une source de rayonnement électromagnétique pour la dégradation photochimique de la toxine.

16. Kit selon la revendication 15, dans lequel le rayonnement électromagnétique comprend un rayonnement ultraviolet.

17. Kit selon l'une quelconque des revendications 1 à 16, dans lequel la composition comprenant ladite toxine botulique pour l'administration transdermique comprend en outre du dioxyde de titane.

18. Kit selon l'une quelconque des revendications 5 à 17, dans lequel le colorant est au moins un sélectionné parmi le groupe constitué de : un colorant artificiel, un colorant alimentaire naturel, FD&C Bleu n° 1, FD&C Bleu n° 2, FD&C Vert n° 3, FD&C Rouge n° 40, FD&C Rouge n° 3, FD&C Jaune n° 5, FD&C Rouge n° 6, colorant caramel, rocou, carmin, bétanine, curcumine, safran, paprika, pandan, et clitoria du Maryland.

19. Kit selon l'une quelconque des revendications précédentes, dans lequel la toxine botulique est présente dans la composition pour fournir une dose de toxine botulique de 100 U à 7 000 U.

20. Kit selon la revendication 19, dans lequel la toxine botulique est présente dans la composition pour fournir une dose de toxine botulique de 500 U à 6 000 U.

21. Kit selon la revendication 20, dans lequel la toxine botulique est présente dans la composition pour fournir une dose de toxine botulique de 1000 U à 5 000 U.

22. Kit selon la revendication 21, dans lequel la toxine botulique est présente dans la composition pour fournir une dose de toxine botulique de 2000 U à 4 000 U.
